# EUROPEAN PATENT SPECIFICATION

(11) **EP 1 667 715 B1**
(45) Date of publication and mention of the grant of the patent: **23.09.2009**
(21) Application number: 04743671.2
(22) Date of filing: 05.08.2004
(51) Int. Cl.: A61K 39/385, C12N 15/62, G01N 33/68, C12Q 1/68

(54) **ANTIGEN DELIVERY SYSTEM**
ANTIGEN-ZUFUHRSYSTEM
SYSTEME DE DELIVRANCE D'ANTIGENES

(30) Priority: 05.08.2003 GB 0318247
(43) Date of publication of application: 14.06.2006
(73) Proprietor: The Royal Veterinary College, London NW1 0TU (GB)
(72) Inventor: WERLING, Dirk The Royal Veterinary College, Hatfield Herts AL9 7TA (GB)
(74) Representative: Miles, John Stephen
(86) International application number: PCT/GB2004/003386
(87) International publication number: WO 2005/014040

(56) References cited:
- WO-A-01/64752
- WO-A-02/20050
- WO-A-03/040169
- WO-A-20/04092195
- US-A1- 2002 187 131

## Description

The present invention relates to an antigen delivery system. In particular, it relates to a system that can be used to vaccinate animals against diseases and to discriminate between vaccinated and naturally infected animals.

Understanding and selective manipulation of the natural innate and adaptive immune system in animals is highly favourable both from a view of protecting animals, both farm animals as well as companion animals, from disease and from a decrease in reliance on antibiotic intervention for disease control due to the increasing risks of antibiotic resistance, a topic of increasing importance in all species.

Antibiotics are frequently utilised on an indiscriminate basis for protection of agricultural and companion animal species against a wide range of pathogens. This culture of inappropriate antibiotics use has developed through poor performance of pathogen antigens with respect to protective immunity.

Therefore, the need to develop and improve our understanding of the role of host adaptive immunity in pathogen protection is being driven, for example, both by animal welfare concerns and the increasing requirements of consumers for high quality meat with reduced antibiotic residues.

There is now an increasing research on molecular antigens with the aim of inducing protection in animal species by their use in vaccine products. However, because such products rarely take into account the requirements for priming of the cellular immune system with regard to protection of animals from disease, these have had limited efficacy in many cases.

One of the limitations of vaccination as a means of disease control is the difficulty of distinguishing naturally infected animals and vaccinated animals. For example, this was a major issue in relation to the recent foot and mouth disease (FMD) outbreak in the UK, where vaccination was not used partly for the reason that once a national cow herd is vaccinated, certain countries will not allow imports of cattle from the herd. Present vaccination methods also lowers the market value of the meat as the carcass has to be treated as if it is infected, and left to hang for longer to kill any virus that may be present. For FMD the emphasis is on keeping clean herds, but it is almost impossible to keep clean herds as transmission is a problem (eg from rats, badgers etc). Marker vaccines, which contain an immunogen foreign to the vaccinated animal, have been proposed as a way of distinguishing naturally-infected and vaccinated animals.

US 2002/0187131 (D2) discloses methods for enhanced antigen delivery to antigen-presenting cells such as dendritic cells and modulation of the immune response therefrom.

I propose that targeting dendritic cells using a moiety which selectively binds to a dendritic cell, in combination with an antigen such as one relevant to an animal disease, and which moiety which selectively binds as said is not naturally occurring in that animal species, provides not only a new concept of antigen delivery (vaccination), but also allows the discrimination between vaccinated and naturally infected animals.

A first aspect of the invention provides a compound for vaccination of an animal comprising (i) a moiety selected from HIV gp120, the LAM protein of *Mycobacterium tuberculosis* or a glycoprotein of Ebola virus, or a part thereof which binds to DC-SIGN on a dendritic cell and is greater than 10 amino acids in length; and (ii) an antigen.

The moiety binds a dendritic cell but does not substantially bind to other types of antigen presenting cells. Whether a moiety selectively binds as said can be determined by measuring binding of the moiety to dendritic cells compared to binding to other antigen presenting cells, for example using fluorescently labelled binding moiety and measuring the fluorescence associated with dendritic cells and other antigen presenting cells. Alternatively, an anti-moiety antibody can be used to determine which cells the moiety binds to in order to determine its selectivity for dendritic cells.

Dendritic cells are highly potent antigen presenting cells and have been shown to be effective as a physiological adjuvant for eliciting prophylactic or therapeutic immunity. They capture microorganisms that enter peripheral mucosal tissues and then migrate to secondary lymphoid organs, where they present these micro-organisms in antigenic form to resting T cells and thus initiate adaptive immune responses.

It is known that dendritic cells have present on their surface proteins, typically receptors, which are selectively expressed and which may be used as a target for a dendritic cell. Thus, the moiety binds to a receptor which itself is selectively expressed on dendritic cells. Typically, a receptor which is selectively expressed on a dendritic cell is one which is present at a level 10-fold, preferably 100-fold, or more preferably 1000-fold higher on dendritic cells than other antigen presenting cells.

The moiety binds to the pattern recognition receptor DC-SIGN on a dendritic cell. A pattern precognition receptor is a receptor that binds a pathogen associated molecular pattern.

DC-SIGN is also known as the cell-specific intercellular adhesion molecule 3-grabbing nonintegrin C-type lectin, DC-SIGN, and is highly expressed on the surface of dendritic cells. DC-SIGN is also sometimes called CD209.

DC-SIGN is a type II transmembrane protein which forms an N-teeminal cytoplasmic domain, a transmembrane domain, a neck region, a tandem repeat region and a C-type (calcium dependent) mannose-binding lectin domain on its C-terminus. In human and mouse DC-SIGN is expressed by dendritic cells present in the dermis, lamina propria of mucosal tissues such as rectum, uterus and cervix, and in the T-cell area of tonsils, lymph nodes and spleen. In humans DC-SIGN has 404 amino acids.

DC-SIGN has been found in human, chimp, gorilla, macaca, and where the cDNA encoding the polypeptide has been cloned. The cDNA and amino acid sequences for DC-SIGN are found in the following GenBank Accession Nos AF391086 (*Macaca mulatta*), AY078913 (*Pan troglodytes*), NM_021155 (Human) and NM_133238 (*Mus*). Figure 1 shows a partial cDNA sequence of one variant of bovine DC-SIGN. The deduced amino acid sequence of variant 1 bovine DC-SIGN, aligned with DC-SIGNs from chimp, macaca and human, is shown in Figure 3. Figure 6 shows a partial cDNA sequence of a second variant of bovine DC-SIGN. The deduced amino acid sequence of variant 2 bovine DC-SIGN, aligned with mouse, human and variant 1 bovine DC-SIGN, is shown in Figure 7. (See Example 7 for details of the cDNA cloning).

A SMART protein domain analysis of human, mouse and bovine (variant 2) DC-SIGN showed that the proteins share structural homology, each containing a C-type lectin receptor motif. In human DC-SIGN, the putative C- lectin domain is between residues 256 and 378; in mouse, the putative C-lectin domain is between residues 108 and 229; and in bovine (variant 2) DC-SIGN, the putative C- lectin domain is between residues 129-248.

DC-SIGN is believed to be selectively expressed on the surface of dendritic cells and because of its role in the immune system is believed to be conserved between species. Thus, for example, the cDNA encoding the amino acid sequences of human and mouse DC-SIGN share 44% sequence similarity when assessed by the computer program described in the figure legends.

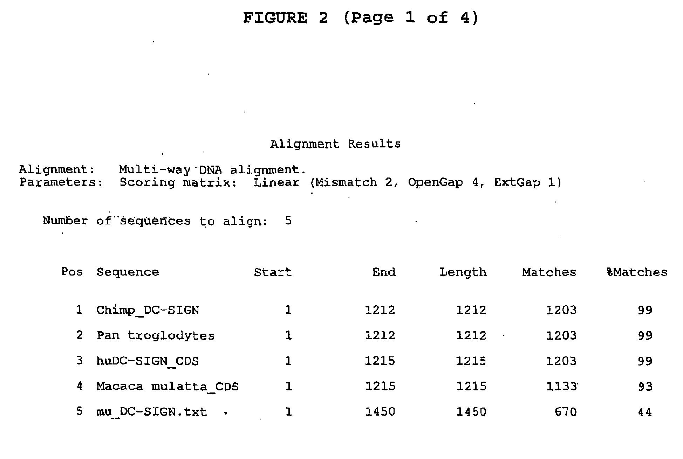

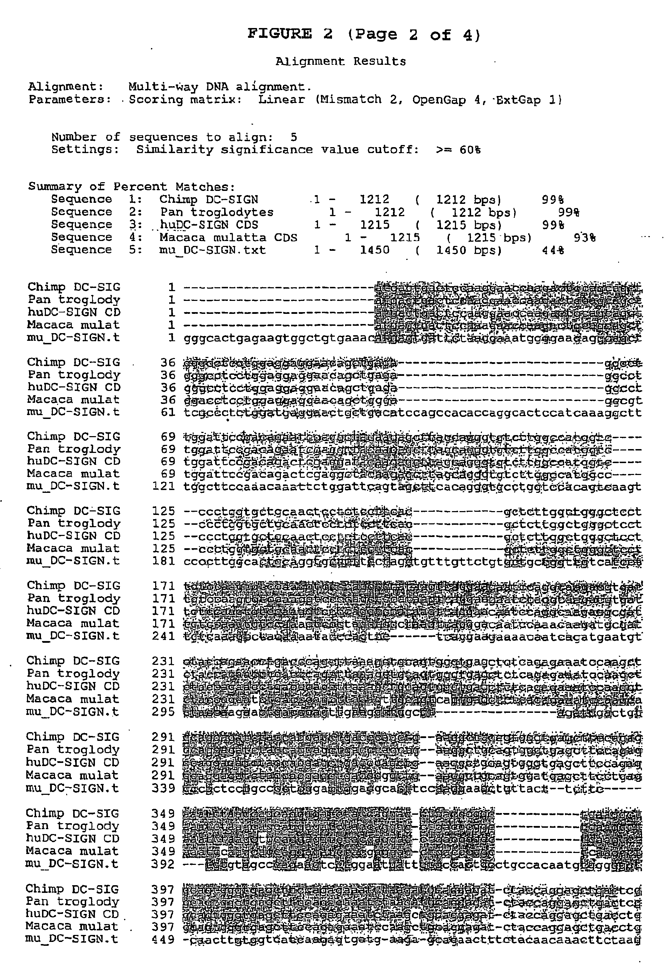

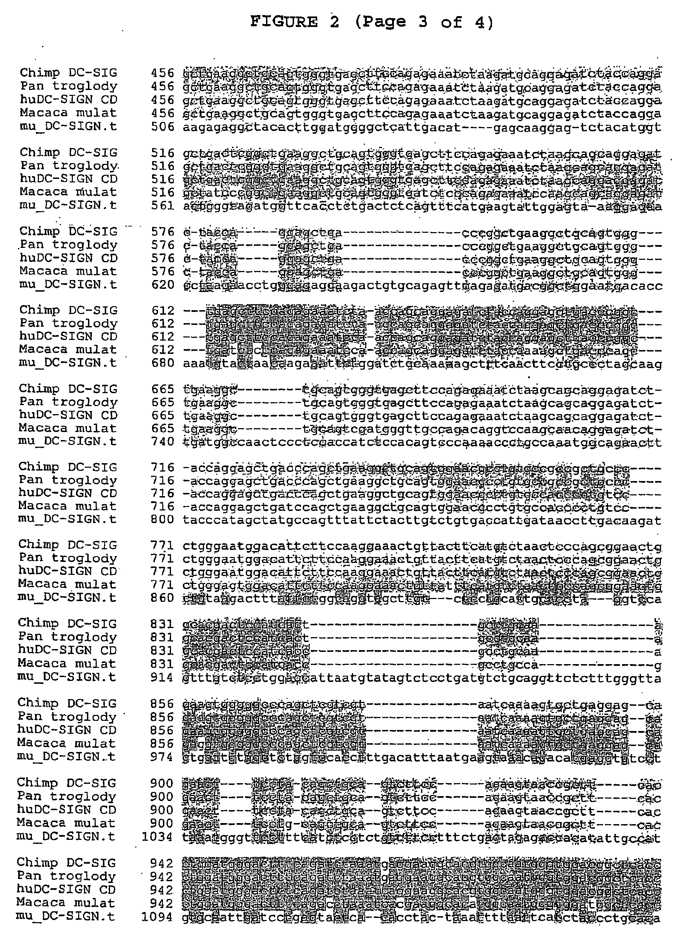

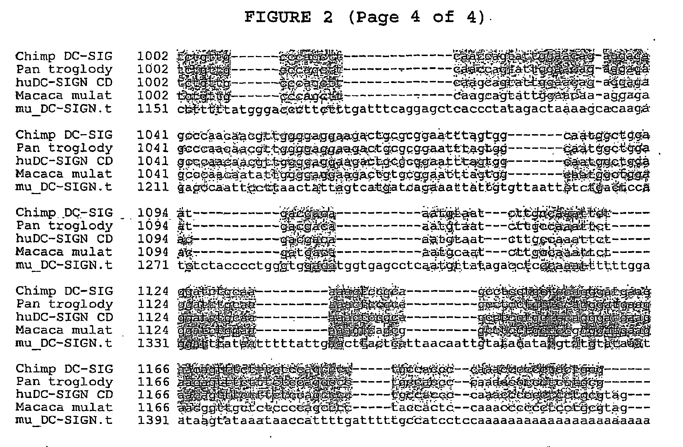

Thus, by DC-SIGN I include any protein whose cDNA encoding the polypeptide sequence has at least 40% similarity with the human or mouse nucleotide sequence shown in Figure 2.

In addition, the DC-SIGN molecule is typically one which is able to bind ICAM-3 or other members of the ICAM family as can be determined, for example, by assays described in Geijtenbeek et al (2002) J. Biol. Chem. 277, 11314-11320.

The following articles describe the interaction between DC-SIGN and HIV-1 or ICAM-3: Geijtenbeek et al (2002) J. Leukoc. Biol. 71, 921-931; Geijtenbeek et al (2002) J. Biol. Chem. 277, 11314-11320; Geijtenbeek et al (2000) Cell 100, 575-585; and Kooyk & Geijtenbeek (2002) Immunol. Rev. 186, 47-56.

In addition, I include any protein whose polypeptide is encoded by a polynucleotide which hybridises under stringent conditions to the polynucleotide whose sequence is given in any one of GenBank Accession Nos AF391086, AY078913, NM_021155 or NM_133238.

By "stringent conditions" I mean hybridising at 6xSSC and 60°C for at least 1 hour, and subsequent washing in 2xSSC at 60°C for 30 minutes. 1xSSC is 0.15M NaCl/0.015M sodium citrate.

Also I include any protein which is immunologically cross-reactive with human or mouse DC-SIGN. Typically, this may be assessed using polyclonal antisera to human or mouse DC-SIGN.

The moiety that selectively binds to a dendritic cell is a protein.

The compound is typically used for vaccination of an animal. Typically, the moiety that selectively binds is all or part of a molecule exogenous or foreign to the animal. It may be from another animal, such as man, and is one which is immunogenic in, and gives rise to an antibody response in, the animal. Thus, it will be seen that not only does the moiety that selectively binds as said target the antigen to the dendritic cell, it will itself also give rise to an immune (antibody) response which serves as a marker of vaccination. In addition, the moiety which selectively binds may also act in stimulating the immune system, typically by priming a Th1 response, and so acts as an adjuvant.

Various moieties have been shown to bind DC-SIGN including the LAM protein of *Mycobacterium tuberculosis* (see, for example, Tailleux et al (2003) J. Exp. Med. 197, 1-5), a glycoprotein of Ebola virus and the HIV-1 envelope glycoprotein gp120 (see, for example, Geijtenbeek et al (2002) J. Biol. Chem. 277, 11314-11320). It is preferred if the binding moiety is able to bind to DC-SIGN with high affinity. It will be appreciated that only a portion of these molecules are required to effect binding to a dendritic cell. Such portions are included in the term "a moiety which binds a dendritic cell". It is preferred, however, if all or substantially all of the molecule is used as the binding moiety.

It is particularly preferred if the moiety that selectively binds to a dendritic cell is HIV-1 envelope glycoprotein gp120. Under natural occurring infections, DC-SIGN mediates HIV transfer by dendritic cells from mucosal surfaces (site of exposure to HIV) to secondary lymphoid organs (site of infection by HIV). This transfer can take several days during which HIV is protected from degradation and retains its infectivity. One potential mechanism is through endocytosis of HIV upon binding DC-SIGN only to return to the cell surface after arrival of the dendritic cell at the lymph node and interaction with T-cells. This internalization hypothesis is supported by the presence of two potential endocytosis motifs, LL and YXXL, in the cytoplasmic domain of DC-SIGN. These motifs have been shown to mediate endocytosis and recycling in various contexts. HIV-1 bound to DC-SIGN transiently expressed in 293T-cells retains its infectivity for several days but is susceptible to trypsin treatment. This may point to the inability of DC-SIGN-expressing 293T-cells to endocytose HIV-1. However, it is possible for HIV-1 internalization to be a significant mechanism of HIV-1 protection in dendritic cells.

The LL and YXXL motifs are conserved between species, as the homologue of the human DC-SIGN molecule has been found in mouse to contain these motifs.

As the initial binding of HIV-1 is mediated by its gp120 envelope protein to DC-SIGN expressed on dendritic cells and given the importance of DC-SIGN in HIV uptake, transport and transmission to T-cells, antigens linked to the gp120 molecule may be used to directly target dendritic cells with a very high efficiency, thus providing an example of the new vaccination approaches described herein.

Thus, targeting DC-SIGN using HIV gp120 protein would: (a) stimulate the host innate immune response by targeting the most potent antigen presenting cells and thus potentially limiting the number of application of the vaccine, the amount needed per vaccination, as well as eliminating the fear of either antibiotic residues or the outgrow of resistant bacterial strains; and (b) enabling the discrimination of vaccinated and naturally infected animals by analysing the antibody response to the HIV gp120 molecule, a protein not naturally occurring in the vaccinated animal, using commercially available test systems.

The parts of the moiety of the invention are able to bind to DC-SIGN on a dendritic cell. The part thereof is greater than 10 amino acid residues in length, more typically greater than 15, 20, 25, 30, 40, 50, 60 or 70 amino acid residues in length.

Although the mycobacterial LAM protein binds DC-SIGN and so may be useful in the practice of the invention, it will be appreciated that some free-ranging animals such as cattle, sheep, horses, goats, deer and so on are in contact with mycobacteria and so may be infected with them. Accordingly, it is preferred that the mycobacterial LAM protein is not used as the moiety which binds to a dendritic cell when the animal is a free ranging animal (or other animal which may be infected with mycobacterium).

Preferably, the compound is one which is endocytosed by the dendritic cell following binding to said cell.

By "antigen" I include any moiety which can elicit an immune response, whether humoral or cell mediated, for example through the production of antibodies, and CD8-mediated, and NK cell-mediated responses. The antigen may refer to an individual molecule or to a homogeneous or heterogeneous population of antigenic molecules. Various macromolecules can act as antigens, including all proteins (if present in the correct context), including nucleoproteins, lipoproteins, most polysaccharides (especially large polysaccharides), and various small molecules (usually called haptens) if they are attached to proteins or polypeptides or other carriers. The term "antigen" also includes antigenic molecules that are multivalent, having multiple epitopes, or monovalent, having only one epitope.

In the context of vaccine production, the antigen is a molecule, or part thereof, or variant thereof, associated with a disease. In particular, in the context of an animal vaccine the antigen is a molecule, or part thereof, associated with a disease of the animal to be vaccinated. Antigens are associated with diseases involving a pathogen, particularly infectious diseases, and it is particularly preferred if the antigen is an antigenic or immunogenic portion of a component of a pathogen, such as an infectious microorganism.

Pathogens include those associated with the following diseases: foot and mouth disease, swine vesicular disease, peste des petits ruminants, lumpy skin disease, bluetongue, African horse sickness, classical swine fever, Newcastle disease, vesicular stomatitis, rinderpest, contagious bovine pleuropneumonia, Rift Valley fever, sheep pox and goat pox, African swine fever and highly pathogenic avian influenza. These are transmissible diseases that have the potential for very serious and rapid spread, irrespective of national borders, that are of serious socio-economic or public health consequence and that are of major importance in the international trade of animals and animal products and are List A diseases of OIE (Office International des Epizooties; www.oie.int).

Pathogens also include those associated with the following: Multiple species diseases including anthrax, Aujeszky's disease, echinococcosis/hydatidosis, heartwater, leptospirosis, new world screwworm (*Cochliomyia hominivorax*), old world screwworm (*Chrysomya bezziana*), paratuberculosis, Q fever, rabies, trichinellosis; cattle diseases including bovine anaplasmosis, bovine babesiosis, bovine brucellosis, bovine cysticercosis, bovine genital campylobacteriosis, bovine spongiform encephalopathy, bovine tuberculosis, dermatophilosis, enzootic bovine leukosis, haemorrhagic septicaemia, infectious bovine rhinotracheitis/infectious pustular vulvovaginitis, malignant catarrhal fever, theileriosis, trichomonosis, trypanosomosis (tsetse-borne); sheep and goat diseases including caprine and ovine brucellosis (excluding *B. ovis*), caprine arthritis/encephalitis, contagious agalactia, contagious caprine pleuropneumonia, enzootic abortion of ewes (ovine chlamydiosis), maedivisna, Nairobi sheep disease, ovine epididymitis (*Brucella ovis*), ovine pulmonary adenomatosis, salmonellosis (*S. abortusovis*), scrapie; equine diseases including contagious equine metritis, dourine, epizootic lymphangitis, equine encephalomyelitis (Eastern and Western), equine infectious anaemia, equine influenza, equine piroplasmosis, equine rhinopneumonitis, equine viral arteritis, glanders, horse mange, horse pox, Japanese encephalitis, surra (*Trypanosoma evansi*), Venezuelan equine encephalomyelitis; swine diseases including atrophic rhinitis of swine, enterovirus encephalomyelitis, porcine brucellosis, porcine cysticercosis, porcine reproductive and respiratory syndrome, transmissible gastroenteritis; and lagomorph diseases including myxomatosis, rabbit haemorrhagic disease and tularemia.

These are some of the List B diseases of OIE; and are transmissible diseases that are considered to be of socio-economic and/or public health importance within countries and that are significant in the international trade of animals and animal products.

Particular pathogens include foot and mouth disease virus, FeLV (feline leukaemia virus), FIV (feline immunodeficiency virus), CDV (canine distemper virus), Parvovirus, coronavirus, bovine respiratory syncytial virus and bovine viral diarrhoea virus.

In addition, however, antigens are associated with diseases which are not necessarily associated with a pathogen including, for example, cancers where tumour antigens are associated with the disease. Typically, tumour antigens are proteins which are abnormally over-expressed in cancer or which are expressed in an abnormal, eg mutant, form in cancer.

Prions or parts thereof are antigens which may be used in the practice of the invention and are not believed to be associated with pathogenic organisms. Prions are associated with spongiform encephalopathies such as bovine spongiform encephalopathy (BSE) and scrapie in sheep.

Vaccine antigens for use in the context of the present invention include antigenic or immunogenic components of microorganisms such as viruses, bacteria, fungus and parasites including helminths, or parts of such components, intended for the prevention of diseases in animals or that provide protection against diseases in animals.

Suitable antigens include, for example, the E2 protein of bovine viral diarrhoea virus (BVDV), the gp51 protein of bovine leukaemia virus, and the F or G proteins of respiratory syncytial virus (RSV). The amino acid sequences for these proteins are encoded in the viral genomes. The GenBank Accession Nos for the viral genomes are: NC_001781 (human RSV), NC 001989 (bovine RSV), AF033818 (bovine leukaemia virus), AF091605 (type I BVDV) and AF145967 (type II BVDV).

Other suitable disease-associated antigens can be selected by the person skilled in the art and typically for many such antigens their amino acid sequence and cDNA sequence encoding them are available in GenBank.

The antigen is typically all or part of a polypeptide associated with a disease such as a polypeptide component of a pathogen or a tumour antigen. The part of the polypeptide may be any part of the polypeptide which is able to elicit an immune response such as an antibody response. It is known that peptides having as few as 5 amino acids may elicit an antibody response, although typically larger peptides are used. Thus, when the antigen is a polypeptide it may have at least 5 amino acids, typically from 5 to 1000 amino acids, such as 5 to 500, 5 to 200, 5 to 100, 5 to 50, 5 to 40, 5 to 30, 5 to 20, for example 5, 6, 7, 8, 9, 10, 11, 12, 13, 14, 15, 16, 17, 18, 19 or 20 amino acids.

It will be appreciated that the antigen of the compound of the invention may be a variant of a molecule associated with a disease provided that it is able to elicit an immune response which is protective against the disease. Such variants include polypeptides which have one or more amino acid substitutions compared to the native antigen associated with the disease, and as many as 5% substitutions. Typically, the substitutions are conservative substitutions where, for example, a "variant" refers to a protein wherein at one or more positions there have been amino acid insertions, deletions, or substitutions, either conservative or non-conservative, provided that such changes result in a protein whose basic properties, for example enzymatic activity (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Gly, Ala; Val, Ile, Leu; Asp, Glu; Asn, Gln; Ser, Thr; Lys, Arg; and Phe, Tyr.

Such variants may be made using standard methods of protein engineering and site-directed mutagenesis.

Preferably the moiety which selectively binds to a dendritic cell as defined in Claim 1 and the antigen are covalently linked. When the moiety and antigen are each a polypeptide, the two portions may be linked together by any of the conventional ways of cross-linking polypeptides. For example, the two portions of the compound of the invention are linked together by any of the conventional ways of cross-linking polypeptides, such as those generally described in O'Sullivan et al Anal. Biochem. (1979) 100, 100-108. For example, the first portion may be enriched with thiol groups and the second portion reacted with a bifunctional agent capable of reacting with those thiol groups, for example the N-hydroxysuccinimide ester of iodoacetic acid (NHIA) or N-succinimidyl-3-(2-pyridyldithio)propionate (SPDP), a heterobifunctional cross-linking agent which incorporates a disulphide bridge between the conjugated species. Amide and thioether bonds, for example achieved with m-maleimidobenzoyl-N-hydroxysuccinimide ester, are generally more stable *in vivo* than disulphide bonds.

Further useful cross-linking agents include S-acetylthioglycolic acid N-hydroxysuccinimide ester (SATA) which is a thiolating reagent for primary amines which allows deprotection of the sulphydryl group under mild conditions (Julian et al (1983) Anal. Biochem. 132, 68), dimethylsuberimidate dihydrochloride and N,N'-o-phenylenedimaleimide.

Alternatively, the compound may be produced as a fusion compound (or fusion polypeptide) by recombinant DNA techniques whereby a length of DNA comprises respective regions encoding the polypeptide portion of moiety that selectively binds to a dendritic cell and the antigen either adjacent to one another or separated by a region encoding a linker peptide which does not destroy the desired properties of the compound.

As used herein, a fusion polypeptide is one that contains a polypeptide which is the polypeptide portion of the moiety which binds a dendritic cell fused at the N- or C-terminal end of a polypeptide which is the antigen of the compound of the invention. A simple way to obtain such a fusion polypeptide is by translation of an in-frame fusion of the polynucleotide sequences, *ie* a hybrid gene. The hybrid gene encoding the fusion polypeptide is inserted into an expression vector which is used to transform or transfect a host cell. Transcriptional and translational control regions are typically present in expression vectors. The DNA is then expressed in a suitable host to produce a polypeptide comprising the compound according to the first aspect of the invention.

It will be appreciated that the antigen may comprise two or more molecules associated with a disease of the animal or parts or variants of such molecules.

In this way, a compound of the invention may be used to vaccinate against more than one disease. Thus, for example, the compound may comprise an antigen from one disease agent (eg the E2 polypeptide of BVDV or an immunogenic portion thereof) and an antigen from a second disease agent (eg the F protein of RSV or an immunogenic agent thereof) in the same polypeptide chain as the moiety which binds a dendritic cell. Other variations will be apparent to the person skilled in the art.

A second aspect of the invention comprises a nucleic acid molecule encoding a fusion compound of the first aspect of the invention.

Suitable nucleic acid molecules may readily be synthesised or constructed by the person skilled in the art using routine methods such as those described in cloning manuals including Sambrook, J. and Russell, D. (2001) "Molecular Cloning: a laboratory manual" 3rd ed. Vol 1-3, Cold Spring Harbor Laboratory Press. Typically the nucleic acid is DNA, but it may be RNA. In the following, where DNA is used, unless the context indicates to the contrary, RNA is also included.

DNA sequences coding for the antigen of the compound of the first aspect of the invention can be identified by searching a database of DNA sequences, such as GenBank and the like. Examples of DNA coding sequences for suitable antigens are given above. Once a DNA sequence coding for the chosen antigen is known, it can be used to design primers and/or probes that are useful in the specific isolation of a DNA or cDNA sequence coding for the antigen, for example from the pathogen associated with the disease to be combated. If a DNA sequence is known, primers and probes can be designed using commercially available software and synthesised by automated synthesis. In general, a DNA sequence coding for the antigen can be isolated from a library of cDNA or DNA sequences generated from an appropriate source, such as the selected pathogen. The library can be screened for the DNA sequences of interest using a probe complementary to a known DNA sequence encoding a selected antigen, preferably under high stringency conditions. DNA sequences that hybridise to the probe can be subcloned and the polypeptide encoded by the DNA sequence can be confirmed by DNA sequence analysis and/or by *in vitro* translation, expression and detection of the polypeptide or like assay. Typically, however, suitable DNA sequences encoding the antigen may be synthesised using the PCR and suitable primers directed at the 5' and 3' ends of the coding region as is well known in the art.

Once the DNA sequence coding for the selected antigen is isolated, it can be operably linked to the DNA sequence encoding the moiety which binds to a dendritic cell as defined in Claim 1, for example HIV gp120 which selectively binds to DC-SIGN of dendritic cells. The DNA sequence encoding the antigen and the moiety which binds to a dendritic cell are both in the same reading frame operably linked to transcriptional and translational control regions. Transcriptional and translational control regions include promoters, enhancers, cis regulatory elements, polyadenylation sequences, transcriptional and translational initiation regions, and transcriptional termination sequences.

The DNA is then expressed in a suitable host to produce a polypeptide which is a compound of the first aspect of the invention. Thus, the DNA encoding the polypeptide constituting the compound of the invention may be used in accordance with known techniques, appropriately modified in view of the teachings contained herein, to construct an expression vector, which is then used to transform an appropriate host cell for the expression and production of the polypeptide of the invention.

Thus, the DNA encoding the polypeptide constituting the compound of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example *E. coli* and *Bacillus subtilis*), yeasts (for example *Saccharomyces cerevisiae*), filamentous fungi (for example *Aspergillus*), plant cells, animal cells and insect cells.

The vectors include a prokaryotic replicon, such as the ColE1 *ori*, for propagation in a prokaryote, even if the vector is used for expression in other, non-prokaryotic, cell types. The vectors can also include an appropriate promoter such as a prokaryotic promoter capable of directing the expression (transcription and translation) of the genes in a bacterial host cell, such as *E*. *coli*, transformed therewith. Typically, the vector is one which may be stably integrated in a host cell and/or gives high expression of the compound. A suitable vector includes pcDNA 3.1 (+/- His tag) available from Invitrogen.

By "promoter" I mean an expression control element formed by a DNA sequence that permits binding of RNA polymerase and transcription to occur. Promoter sequences compatible with exemplary hosts are typically provided in plasmid vectors containing convenient restriction sites for insertion of a DNA segment of the present invention.

Another aspect of the invention therefore provides a host cell transformed with a nucleic acid encoding the compound according to the first aspect of the invention.

Particularly preferred host cells for expression of the compound of the invention include COS and CHO cells which are able to glycosylate proteins. *E. coli* cells may be used and in that case, the lipopolysaccharide component which may be present in the final product of the compound may in itself be an immunostimulant. This may be beneficial in some circumstances, but may be undesirable in others where it may compromise the measurement of an immune response.

Culturing of the host cell under conditions permitting expression of the DNA polymer is carried out conventionally. The product may be recovered by conventional methods according to the host cell and according to the localisation of the expression product (intracellular or secreted into the culture medium or into the cell periplasm). Thus, where the host cell is bacterial, such as *E*. *coli* it may, for example, be lysed physically, chemically or enzymatically and the protein product isolated from the resulting lysate. Where the host cell is mammalian, the product may generally be isolated from the nutrient medium or from cell free extracts. Where the host cell is a yeast such as *Saccharomyces cerevisiae* or *Pichia pastoris*, the product may generally be isolated from lysed cells or from the culture medium, and then further purified using conventional techniques. The specificity of the expression system may be assessed by western blot using an antibody directed against the polypeptide of interest.

Conventional protein isolation techniques include selective precipitation adsorption chromatography, and affinity chromatography including a monoclonal antibody affinity column.

It will be appreciated that the invention can be practised using a "cassette expression" vector which contains a coding sequence for the moiety which binds a dendritic cell, such as HIV-1 gp120, which can readily be fused to a coding region for the chosen antigen which is inserted at an appropriate place in the cassette expression vector.

Typically, the nucleic acid molecule takes the form of an expression vector which contains appropriate transcriptional and translational control signals to allow expression of the fusion polypeptide once the antigen coding region has been inserted into the insertion point. As is well known in the art, the insertion point in a "cassette expression" vector is one which readily allows for the insertion of the appropriate coding sequence (one encoding an antigen in this case) so that an inframe fusion is produced. Typically, the insertion point is a unique restriction site within the nucleic acid.

Typically, the vector is one which may be stably integrated in a host cell and/or gives a high level of expression of the compound. It may, conveniently be based on the pcDNA3.1 (+/- His tag) vector available from Invitrogen.

Since the antigen may be fused to the binding moiety at its N-terminus or C-terminus, the insertion point may be at the 5' end or 3' end of the coding sequence for the binding moiety, and suitable transcription and translation signals placed appropriately as will be known to the person skilled in the art.

The compound of the first aspect of the invention is useful in the immunisation against, and treatment of, diseases in animals. Thus, the compound is useful in a vaccine.

A further aspect of the invention provides a compound according to the first aspect of the invention for use in medicine. Typically, the compound is packaged and presented as a medicament for use in an animal.

A still further aspect of the invention provides a vaccine comprising a compound according to the first aspect of the invention is preferably adjuvanted in the vaccine formulation of the invention. Suitable adjuvants are commercially available such as alum and Quil A.

It is preferably that the adjuvant composition induces an immune response predominantly of the Th1 type. High levels of Th1-type cytokines (for example, IFN-γ, TNFα, IL-2 and IL-12) tend to favour the induction of cell mediated immune responses to an administered antigen. Preferably, the compound may give rise to both a Th1 and Th2 responses.

A still further aspect of the invention provides a pharmaceutical composition comprising a compound according to the first aspect of the invention and a pharmaceutically acceptable carrier.

The carrier(s) must be "acceptable" in the sense of being compatible with the compound of the invention and not deleterious to the recipients thereof. Typically, the carriers will be water or saline which will be sterile and pyrogen free. However, other acceptable carriers may be used.

Typically, the pharmaceutical compositions or formulations of the invention are for parenteral administration, more particularly for intravenous administration. The compositions or formulations may be administered by the following routes: intramuscular, subcutaneous, intradermal, intranasal, intravenous, oral, and intraperitoneal. Intramuscular is most preferred as it is the most practical way for a vet.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The compositions may be directed towards immunising and protecting animals, preferably animals of economic importance, including farm animals such as cows, sheep, goats, pigs, horses and rabbits, and companion animals, such as cats and dogs. Depending on the type of antigens used, immunisation of animals with these antigens can result in the prevention of infection, amelioration of symptoms, decrease in mortality and/or induction of neutralising antibodies.

Diseases which may be immunised against or combated using the compounds of the invention include BVDV, RSV and bovine leukaemia virus and appropriate antigens may be selected for example from the BVDV E2 protein, the RSV F and G proteins and gp51 of bovine leukaemia virus. Thus, the compound of the invention may be used prophylactially or therapeutically.

Typically, the disease to be treated is one caused by a pathogen. However, tumours may also be treated.

The tumours that may be treated by the compounds of the invention include melanomas (see, for example, Nestle, F.O. (2002) Clinical & Experimental Dermatology 27(7), 597-601). Typically, more than one antigen may be needed in order to be effective. These may be combined in the same compound for vaccination or in separate such compounds. Typically, the antigens may be mutated antigens (eg mutated p16 (CDKN2A)), shared tumour specific antigens (eg MAGE-1, MAGE-3 and NY-ESO-1), differentiation antigens (eg tyrosinase, gp100 and MelanA/MART-1) or cell surface gangliosides (eg GM2, GD2 and GD3) as described in Nestle (2002) *supra.*

The term "tumour" is to be understood as referring to all forms of neoplastic cell growth, including tumours of the lung, liver, blood cells, skin, pancreas, stomach, colon, prostate, uterus, breast, lymph glands and bladder. Solid tumours are especially suitable.

Still further aspects of the invention provide the use of a compound according to the first aspect of the invention in the manufacture of a medicament for combating a disease in an animal; or in the manufacture of a vaccine for immunising an animal.

Particularly preferred embodiments include the use of a compound comprising (i) a moiety which selectively binds to a dendritic cell in an animal but which does not naturally occur in said animal as defined in Claim 1 and (ii) an antigen relevant to a disease in said animal in the manufacture of a medicament for combating the disease in the animal; and the use of a compound comprising (i) a moiety which selectively binds to a dendritic cell in an animal but which does not naturally occur in said animal as defined in Claim 1 and (ii) an antigen relevant to a disease in said animal in the manufacture of a vaccine for immunising the animal.

The invention particularly includes the use of a compound for vaccination of an animal comprising (i) a moiety selected from HIV gp120, the LAM protein of *Mycobacterium tuberculosis* or a glycoprotein of Ebola virus, or parts thereof as defined in Claim 1, and (ii) an antigen in the manufacture of a medicament for combating disease associated with the antigen or in the manufacture of a vaccine for immunising the animal against the disease associated with the antigen.

Animals vaccinated using the compounds of the invention can be distinguished from animals naturally infected with the disease agent with which the antigen of the compound of the invention is associated since vaccinated animals will have had an immune response to the moiety which binds to a dendritic cell whereas naturally-infected animals will not.

Thus, a further aspect of the invention provides a method of determining whether an animal has been administered a compound according to the first aspect of the invention, the method comprising determining in an antibody-containing sample taken from the animal whether the animal has had an immune response to the moiety in the compound as defined in Claim 1. It may also be useful to determine whether the animal has had an immune response to the antigen present in the compound.

Typically, an antibody-containing sample is taken from the animal and it is determined whether an antibody directed at the said moiety is present. Whether an antibody directed at said antigen is present may also be determined. Typically, the sample is a blood sample. Typically, the blood sample is obtained from the jugular vein or tail vein of the animal.

The invention also provides kits of parts which may be used to distinguish naturally-infected animals and those administered a compound of the first aspect of the invention.

One kit of parts comprises (i) a compound according to the first aspect of the invention and (ii) means for detecting an immune response to the moiety present in the compound as defined in Claim 1 and/or (iii) means for detecting an immune response to the antigen in said compound.

A further kit of parts comprises (i) means for detecting an immune response to an animal disease antigen and (ii) means for detecting an immune response to a moiety selected from HIV gp120, the LAM protein of Mycobacterium tuberculosis or a glycoprotein of Ebola virus, or a part thereof which binds to DC-SIGN on a dendritic cell and is greater than 10 amino acids in length.

In a preferred embodiment, the binding moiety is HIV-1 ge120, or a part thereof greater than 10 amino acids in length.

Typically, an immune response is detected by determining whether or not a sample from the animal contains appropriate antibodies (ie to the antigen and/or the binding moiety).

Thus, suitable means for detecting the immune response or responses include using an ELISA to determine an antibody response. An ELISA for measuring gp120 antibody response are commercially available, for example from Advanced BioSciences laboratories Ltd or ImmunoDiagnostics Inc.

The invention will now be described in more detail by reference to the following Figures and Examples.

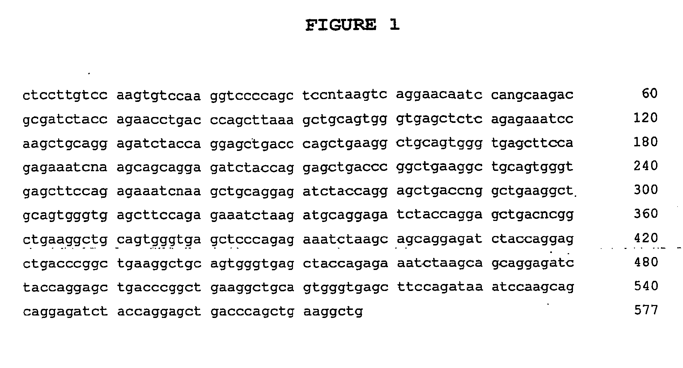

Figure 1 shows the partial cDNA sequence encoding bovine DC-SIGN variant 1 (SEQ ID No. 1). The letter "n" means any of the four naturally occurring nucleotides A, G, C or T.

Figure 2 shows the alignment of cDNA sequences encoding DC-SIGN from chimp (*Pan troglodytes*), human, Macaca and mouse (SEQ ID Nos. 2-5, respectively) using the SE Central sequence analysis package (Align Plus and Clone Manager) from Scientific and Educational Software.

The alignment, parameters and settings are indicated. Dashes indicate no nucleotides.

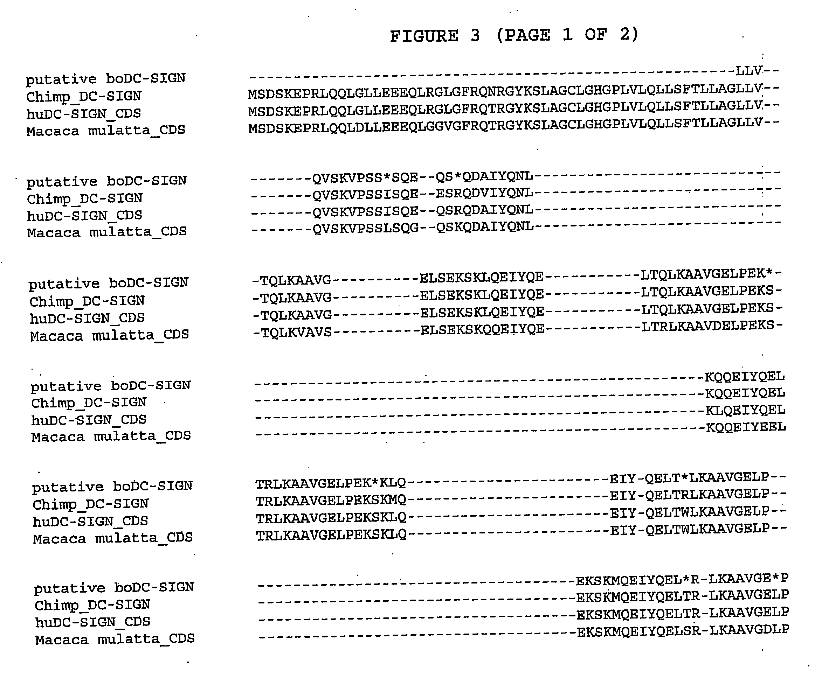

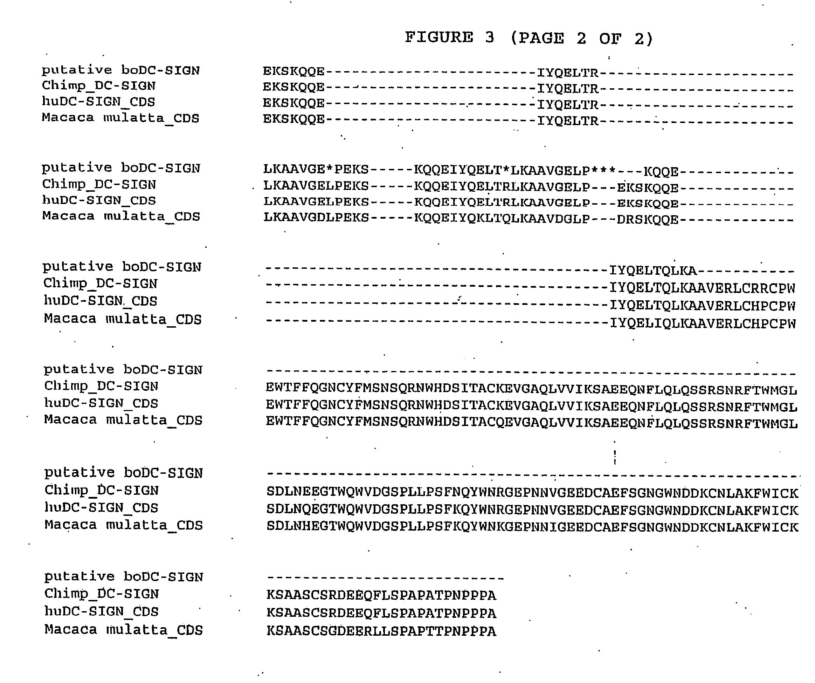

Figure 3 shows the alignment of the deduced partial amino acid sequence for bovine DC-SIGN variant 1 (SEQ ID No. 6) against amino acid sequence for DC-SIGNs from chimp, human and macaca (SEQ ID Nos. 7-9). Dashes indicate no amino acids. The asterisks in the bovine DC-SIGN amino acid sequence are unallocated amino acids. The alignments were done using the SE Central sequence analysis package. The Alignment parameters were: Global Protein alignment against reference molecule; Parameters: Scoring matrix: BLOSUM 62; Reference molecule putative boDC-SIGN, region 1-577; Number of sequences to align 4. Putative boDC-SIGN 192 amino acids; chimp_DC-SIGN 404 amino acids; huDC_SIGN_CDS 404 amino acids; Macaca mulatta_CDS 404 amino acids.

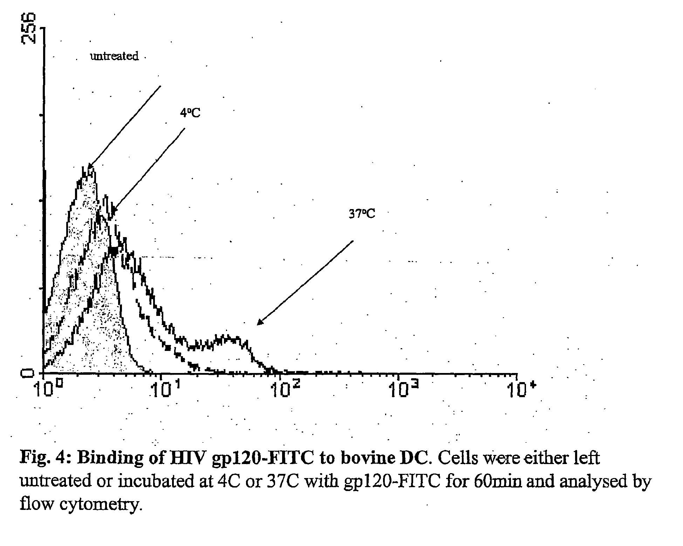

Figure 4 is a graph showing the binding of HIV gp120-FITC to bovine DC. Cells were either left untreated or were incubated at 4°C or 37°C with gp 120-FITC for 60 minutes and analysed by flow cytometry.

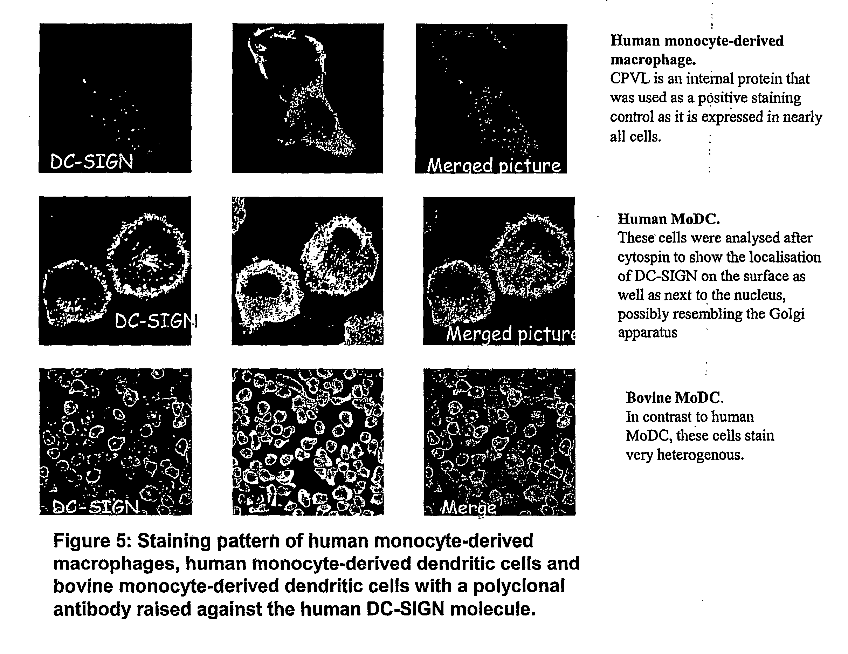

Figure 5 shows the staining pattern of human monocyte-derived macrophages, human monocyte-derived dendritic cells and bovine monocle-derived dendritic cells with a polyclonal antibody raised against the human DC-SIGN molecule.

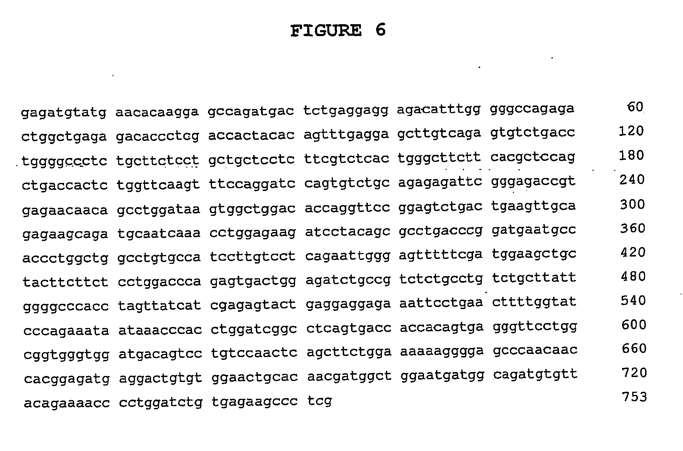

Figure 6 shows the partial cDNA sequence encoding bovine DC-SIGN, variant 2 (SEQ ID No. 10).

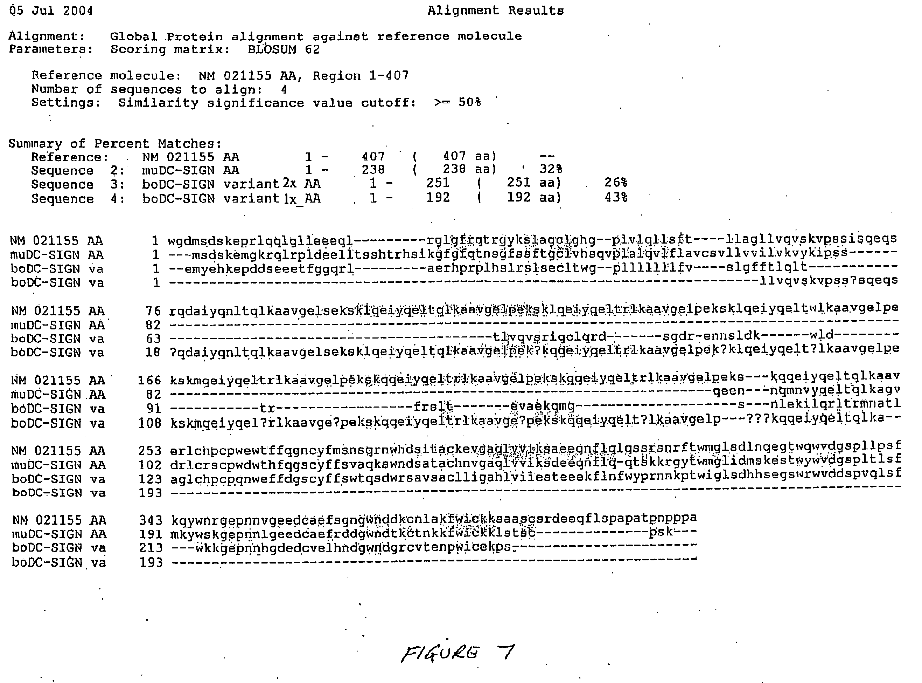

Figure 7 shows a CLUSTAL W (1.7) alignment of the amino acid sequences of human DC-SIGN (SEQ ID No. 11), murine DC-SIGN (SEQ ID No. 12), and the two variants of bovine DC-SIGN (SEQ ID Nos. 6 and 13 (variants 1 and 2, respectively)). Dashes indicate no matching residues.

### Example 1: Ability of gp120 protein to bind to DC-SIGN on bovine cells

I have shown by flow cytometry that HIV gp120-FITC proteins (Cat No 1021-F and 1001-F, Immuno Diagnostic Inc., Woburn, USA) bind to bovine DC. Incubation of HIV gp120-FITC at 4°C for 1 hour induced low level of binding, but this was increased by incubation at 37°C (Figure 4).

Furthermore, a rabbit anti-human polyclonal antibody raised against the human DC-SIGN molecule (kindly provided by Dr. T. Geijtenbeck (Department of Molecular Cell Biology, Medical Faculty, Vrije Universiteit Amsterdam) was shown by laser scanning confocal microscopy to stain bovine DC (Figure 5) to a lesser extent than human DC (Figure 5).

To assess the ability of HIV-1 ge120 protein to bind to DC-SIGN on bovine cells, cDNA encoding the protein is cloned into an expression vector containing a His-tag (ie oligo histidine tag). As a control, an unrelated His-tagged protein that is known not to bind to DC-SIGN, such as FITC-labelled ovalbumin (OVA), is used. To follow the uptake of sp120-His by DC, a DC culture is prepared and divided into 4 groups of cells. First (experimental) and second (control) groups are pulsed with either gp120-His or an unrelated His-tagged protein for 2 hours and washed. The third (negative control) group is only treated with growth medium, whereas the fourth group (uptake control) is used to demonstrate the ability of DC to take up FITC-labelled antigen (in this case OVA) as a functional control for the ability of DC to take up antigen. Subsequently, all groups of cells are used to observe the cellular localisation pattern of proteins by confocal fluorescent microscopy, using a FITC-labelled anti-His antibody. Finally, polyclonal antibodies are raised against the bovine DC-SIGN molecule to show the specificity of gp120-His uptake via DC-SIGN by pre-incubating DC with these antibodies to block DC-SIGN. Blocking of DC-SIGN should reduce uptake of gp120-His, but not any other molecules or the control.

Additionally or alternatively, to assess the ability of the gp120 protein to bind to DC-SIGN on bovine cells, cDNA encoding the protein is cloned into an expression vector containing a His-tag using using RT-PCR and directional cloning approaches using a construct obtained from the NIH AIDS reagent program. The resulting plasmid is used to transfect COS-7 cells, and the expressed protein is concentrated from cell supernatant and/or cell lysates using a molecular weight cut-off spin column or a His-tag purification column. The presence of the gp120-His is assessed by western blotting using anti-His antibodies. To follow the uptake of gp120-His by bovine DC, a DC culture is prepared and divided into 3 groups of cells. First (experimental) and second (control) groups are pulsed with either gp 120-His or supernatant derived from COS-7 cells transfected with the empty plasmid for 2 hours and washed. The third group (uptake control; in this case FITC-OVA) will be used as a functional control to prove the ability of DC to take up antigen (Werling, D et al (1999) J Leukoc Biol 66: 50-58). Subsequently, all groups of cells are used to either observe the amount of protein taken up or to identify the cellular localization of the proteins by flow cytometry and confocal fluorescent microscopy. A FITC-labelled anti-His antibody is used to detect gp120-His. In addition, COS-7 cells, stably transfected with the human DC-SIGN molecule, are used as a positive control.

### Results

Based on the current similarities on published DC-SIGN sequences, it is expected that the gp120-His will bind and subsequently be internalised via the bovine DC-SIGN molecule, and this binding/uptake will be blocked by polyclonal antibodies, whereas this will have no effect on the controls used.

### Discussion

To discriminate between bound and internalised gp120-His, one can measure the amount of gp120-His taken up by the cell, cells will be treated with EDTA-trypsin solution to degrade gp120-His bound on the surface, but not internalised. Thereafter, cellular and cytosolic extracts can be analysed for the presence of gp120-His by western blotting, either using a monoclonal antibody to the His-tag or directly to the gp120 protein. Upon successful uptake of gp120-His, gp 120 is expressed together with model-antigens (*ie* envelope proteins of bovine respiratory syncytial virus or bovine viral diarrhoea virus E2 protein) which will be used for further studies (for the sake of simplicity, this product is termed gp 120-Ag).

### Example 2: Enhanced development of a Th1-type immune response by antigen-pulsed DC

Recent data emphasise that uptake of antigens via pattern recognition receptors, such as Toll-like receptors (TLR) or DC-SIGN, strongly enhances the development of a Th1-type of immune response with IFNα and IL-12 being released by antigen-pulsed DC. The release of these cytokines favours the development of a cell-mediated immunity.

One antigen known to bind to TLR is the F protein of the respiratory syncytial virus (RSV), and this binding leads to the induction of IL-12 (Haeberle, HA et al (2002) J Infect Dis 186: 1199-1206; Haynes, LM et al (2001) J Virol 75: 10730-10737; and Kurt-Jones, EA et al (2000) Nat Immunol 1: 398-401). As RSV is the major cause of lower respiratory tract infections in newborns of several species including cattle and humans, the RSV F protein will be used in these experiments as the model antigen.

His-tagged expression vectors containing HIV gp120 (generated in Experiment 1), the RSV F protein (kindly provided by Dr Geraldine Taylor, Institute for Animal Health, Compton), or a fusion protein of gp 120/F protein (generated in Experiment 1) are used to transfect COS-7 cells. After 72 hours, His-tagged proteins are harvested from the cell supernatant as well as the lysed cells using nickel-agarose, and their presence analysed by western blotting using anti-His-antibodies.

To demonstrate the effect of gp120-His, F-protein-His or gp120-Ag on bovine DC, cells are incubated for 2h with different doses of each purified protein. After this time, the supernatants are harvested and analysed for the presence of IFNα, IL-10 and IL-12 using ELISA-systems (Werling, D et al (2004) Immunology 111: 41-52).

### Results

It is expected that the amount of IFNα and IL-12 released by DC exposed to gp120-His or gp120-Ag should be greater than that produced by peripheral blood lymphocytes or macrophages exposed for the same time and amount to gp120-His. Furthermore, it is expected that gp 120-His alone will result in the production of IL-10, the F-protein-His will result mainly in the production ofIL-12, and the gp120-Ag will result in a very strong release of IL-12.

### Example 3: Stimulation of naïve T-cells by DC

In contrast to other antigen-presenting cells (APC), such as macrophages and B cells, DC have the unique capacity to stimulate naïve T cells and to stimulate a far stronger memory T cell response (Werling *et al* (1999); Werling, D et al (2002) J Leukoc Biol 72: 297-304). To evaluate the stimulatory capacity of gp120-Ag pulsed DC, and whether they are able to stimulate naïve as well as memory T cells, different subsets of APC are generated (Werling *et al* (2002) and incubated with gp120-Ag for different periods of time (0-6h). After this time, DC are inactivated by exposure to mitomycin-D and added in various relations to sorted CD4+ T cells of the same donor. After 5 days in culture, the stimulatory capacity of gp120-Ag pulsed DC, macrophages and B cells are evaluated by measuring the amount of [³H]-labelled thymidine incorporated in the DNA of proliferating T cells by liquid scintillation counting using a beta-counter.

Similar experiments are performed using APC and T cells generated from BRSV-immunized cattle, thus assessing the properties of APC subsets to stimulate a memory T cell response (access to immunized animals exists via Dr Geraldine Taylor, Institute for Animal Health, Compton).

### Results

It is expected that strong T cell proliferation is observed only in the presence of DC, not macrophages or B cells (which show a 10-fold lower response).

It is also expected that only DC pulsed with gp120-Ag will stimulate proliferation of naïve T cells and will induce a proliferative response 10-times stronger than other APC when co-cultured with T cells from BRSV-immunized animals.

### Example 4: Endocytosis of DC-SIGN as a result of HIV-1 binding

In the human system, endocytosis of DC-SIGN as a result of HTV-1 binding decreases the number of DC-SIGN molecules on the DC surface. Specific monoclonal anti-DC-SIGN antibodies are raised in the mouse by standard methods or polyclonal anti-DC-SIGN antibodies are raised in the rabbit by standard methods, and are tested for whether they interfere with gp120-Ag or gp120-His binding to DC-SIGN. A non-interfering antibody and a fluorescent-tagged secondary anti-mouse antibody are used to quantify DC-SIGN expression on the surface of DC (pulsed with gp120-Ag medium or the supernatant of COS-7 cells transfected with an empty plasmid, as negative controls) with the help of Fluorescence Activated Cell Sorting (FACS). The measurement is repeated on a similar group of DC after pulsing with gp120-Ag or gp120-His for 2 hours (experimental group).

Sufficient amount of gp120-Ag should be used to increase the likelihood of DC-SIGN binding and internalisation.

### Results

If the amount of surface DC-SIGN in the experimental group is similar to that of the control group, it is expected that probably no significant endocytosis of DC-SIGN occurs, thus supporting the theory that DC-SIGN bound antigen is protected from degradation, and is not processed. If surface DC-SIGN expression is reduced upon gp120-Ag binding, DC-SIGN is most likely endocytosed by DC, and therefore will be delivered subsequently to endocytic compartments inside the cell, which allow processing of the protein for subsequent presentation to T cells via MHC class II (and MHC class I) molecules. This would results in a subsequent proliferative response of T cells.

It is necessary for endocytosis to occur for an immune response to occur.

Blocking of DC-SIGN should reduce uptake of gp120-His, but not other molecules.

Potentially useful control groups include DC with no surface DC-SIGN, for example as a result of brief trypsin-treatment, and an agent that causes endocytosis of DC-SIGN (positive control).

### Example 5: Effect of ap120-Ag immunisation in animals

To demonstrate the effect of a gp120-Ag immunisation in animals, cattle are immunised with different doses of gp120-Ag, and the development of an antibody response to either an antigen such as the F-protein or gp 120 is analysed using ELISA-systems available for the specific Ag. Suitable ELISA systems are commercially available for gp120 and for the RSV-F protein. Four groups of animals are immunised either with the carrier solution alone, purified gp120-His, purified purified F-protein-His, or gp120-Ag. Blood samples are taken on a weekly base before and after immunisation, and the antibody titre measured in serum samples.

### Results

It is expected that animals immunised with the gp120-Ag will develop antibodies to both the gp120 as well as the Ag of interest, such as the F protein. To estimate the relation between Ag-specific antibodies and gp120-specific antibodies, the antibody-titre of animals immunised with the gp120-His will serve as control, thus establishing data to demonstrate the efficiency of gp120-Ag being taken up and presented.

Typically, the antibody titre to gp 120 in animals immunised with the gp 120-Ag can be used as a direct indicator for the successful immunisation of an individual animal, and will also allow the discrimination of immunised versus naturally infected animals.

### Example 6: Challenging successfully immunised animals with antigen

Having established the successful immunisation of animals, the same groups of animals are challenged with an antigen, such as the RSV F protein (or whole RSV) and the development of clinical signs monitored.

### Results

As the F protein is one of the main antigens of a RSV, it is expected that immunised animals should have a protection against the subsequent challenge and should not develop any clinical symptoms, whereas animals given the gp120-His or carrier solution should do so.

### Example 7: Obtaining bovine DC-SIGN cDNA sequences

Bovine dendritic cells were isolated according to the procedure in Werling et al (2002) J. Leukoc. Biol. 72, 297-304, and mRNA isolated. A SMART™ RACE cDNA amplification kit from Becton Dickinson (Cat. No. K1811-1) was used to amplify cDNA using the primer TAGCTGACTCCTTGTCCAAGTG (SEQ ID NO: 14). The amplified cDNA, referred to as variant 1, was sequenced and the nucleotide sequence is given in Figure 1.

A further partial bovine DC-SIGN cDNA sequence was obtained, referred to as variant 2, using degenerate primers based on homologies shared between the murine and the human DC-SIGN molecule (GenBank Accession Nos AF373408 and NM_021155 respectively). The partial cDNA sequence of variant 2 of bovine DC-SIGN is given in Figure 6.

Both of the variant bovine DC-SIGN cDNA sequences code for a C-type lectin receptor and share up to 89% homology on the base pair and/or amino acid sequence with the corresponding murine and human sequences (Figure 7).

Figure 7 shows a Clustal W alignment of the amino acid sequences of human DC-SIGN (NM_021155_AA), murine DC-SIGN (muDC-SIGN_AA), and the two variants of bovine DC-SIGN (boDC-SIGN variant 1/2_AA). Dashes indicate no matching residues.

## Claims

1. A compound for vaccination of an animal comprising (i) a moiety selected from HIV gp120, the LAM protein of *Mycobacterium tuberculosis* or a glycoprotein of Ebola virus, or a part thereof which binds to DC-SIGN on a dendritic cell and is greater than 10 amino acids in length; and (ii) an antigen.

2. A compound according to Claim 1 wherein the antigen is a polypeptide.

3. A compound according to any one of the preceding claims wherein the antigen is an antigenic component of a pathogen or a tumour, or, a prion antigen.

4. A compound according to Claim 3 wherein the antigen comprises two or more of an antigenic component of a pathogen or a tumour, or, a prion antigen.

5. A compound according to Claim 3 or 4 wherein the antigen is an antigenic variant of an antigenic component having up to 5% substitutions.

6. A compound according to Claims 3 - 5 wherein the pathogen is any of a bacterium, virus, fungus, protozoa or helminth.

7. A compound according to Claim 6 wherein the antigen is an antigenic component of a pathogen associated with foot and mouth disease, swine vesicular disease, peste des petits ruminants, lumpy skin disease, bluetongue, African horse sickness, classical swine fever, Newcastle disease, vesicular stomatitis, rinderpest, contagious bovine pleuropneumonia, Rift Valley fever, sheep pox and goat pox, African swine fever and highly pathogenic avian influenza or a variant of such a component having up to 5% substitutions.

8. A compound according to any one of the preceding claims wherein the moiety and the antigen are covalently linked.

9. A compound according to any one of the preceding claims wherein the moiety and the antigen, each comprise a polypeptide and both are present in the same polypeptide chain.

10. A nucleic acid molecule encoding a compound according to Claim 9.

11. An expression vector comprising a nucleic acid molecule according to Claim 10.

12. A host cell comprising a nucleic acid molecule according to Claim 10 or an expression vector according to Claim 11.

13. A vaccine comprising a compound according to any one of Claims 1 to 9 or a nucleic acid molecule according to Claim 10.

14. A vaccine according to Claim 13 further comprising an adjuvant.

15. A compound according to any one of Claims 1 to 9 or a nucleic acid molecule according to Claim 10 for use in medicine.

16. A pharmaceutical composition comprising a compound according to any one of Claims 1 to 9 or a nucleic acid molecule according to Claim 10 and a pharmaceutically acceptable carrier.

17. Use of a compound according to any one of Claims 1 to 9 or a nucleic acid according to Claim 10 in the manufacture of a medicament for combating a disease in an animal.

18. Use of a compound according to any one of Claims 1 to 9 or a nucleic acid according to Claim 10 in the manufacture of a vaccine for immunising an animal.

19. Use according to Claim 17 or 18 wherein the disease is one caused by a pathogen.

20. Use according to Claim 19 wherein the pathogen is any of a bacterium, virus, fungus, protozoa or helminth.

21. Use according to Claim 20 wherein the pathogen is one associated with foot and mouth disease, swine vesicular disease, peste des petits ruminants, lumpy skin disease, bluetongue, African horse sickness, classical swine fever, Newcastle disease, vesicular stomatitis, rinderpest, contagious bovine pleuropneumonia, Rift Valley fever, sheep pox and goat pox, African swine fever and highly pathogenic avian influenza.

22. Use according to Claim 17 or 18 wherein the animal is a mammal.

23. Use according to Claim 17 or 18 wherein the animal is a companion animal or farm animal.

24. Use according to Claim 22 or 23 wherein the animal is a cow, sheep, horse, pig, goat, dog, cat or rabbit.

25. A method of making a compound according to Claim 1 comprising linking the said moiety and the said antigen.

26. A method of making a compound according to Claim 9 which comprises a polypeptide, said method comprising (i) culturing a host cell according to Claim 12 which expresses said polypeptide and (ii) isolating said polypeptide.

27. A method of making a nucleic acid according to Claim 10 comprising linking a nucleic acid molecule which encodes the moiety and a nucleic acid molecule which encodes an antigen.

28. A method of determining whether an animal has been administered a compound according to Claim 1, the method comprising determining in an antibody-containing sample taken from the animal, whether the animal has had an immune response to the moiety in the compound as defined in Claim 1.

29. A method according to Claim 28 comprising the further step of determining whether the animal has had an immune response to the antigen present in said compound.

30. A kit of parts comprising (i) a compound according to any one of Claims 1 to 9 or a nucleic acid molecule according to Claim 10 and (ii) means for detecting an immune response to the moiety present in said compound and/or (iii) means for detecting an immune response to the antigen present in said compound.

31. A kit of parts according to Claim 30 wherein if present part (ii) comprises all, or a portion greater than 10 amino acid residues in length, of said moiety which binds to an antibody raised against said moiety and part (iii) comprises all, or a portion at least 5 amino acids in length, of said antigen which binds to an antibody raised against said antigen.

32. A kit of parts comprising (i) means for detecting an immune response to an animal disease antigen and (ii) means for detecting an immune response to a moiety selected from HIV gp120, the LAM protein of *Mycobacterium tuberculosis* or a glycoprotein of Ebola virus, or a part thereof which binds to DC-SIGN on a dendritic cell and is greater than 10 amino acids in length.

33. A kit of parts according to any one of Claims 30 to 32 wherein the means for detecting an immune response is an ELISA.

## Patentansprüche

1. Verbindung zum Impfen eines Tiers mit (i) einem Rest, der ausgewählt ist aus HIV gp120, dem LAM-Protein von *Mycobacterium tuberculosis* oder einem Glykoprotein von Ebolavirus oder einem Teil davon, der an DC-SIGN an einer dendritischen Zelle bindet und größer als 10 Aminosäuren in der Länge ist; und (ii) einem Antigen.

2. Verbindung nach Anspruch 1, wobei das Antigen ein Polypeptid ist.

3. Verbindung nach einem der vorangehenden Ansprüche, wobei das Antigen eine antigene Komponente eines Pathogens oder eines Tumors oder ein Prionantigen ist.

4. Verbindung nach Anspruch 3, wobei das Antigen zwei oder mehr aufweist von: einer antigenen Komponente eines Pathogens oder eines Tumors und einem Prion-Antigen.

5. Verbindung nach Anspruch 3 oder 4, wobei das Antigen eine antigene Variante einer antigenen Komponente ist, die bis zu 5 % Substitutionen aufweist.

6. Verbindung nach Anspruch 3 bis 5, wobei das Pathogen ein Bakterium, Virus, Pilz, Protozoon oder Ethelminth ist.

7. Verbindung nach Anspruch 6, wobei das Antigen eine antigene Komponente eines Pathogens, das mit Maul und Klauenseuche, vesikulärer Virusseuche der Schweine, Pest der kleinen Wiederkäuer, Dermatitis nodularis, Blauzungenkrankheit, Afrikanischer Pferdepest, klassischem Schweinefieber, Newcastle-Krankheit, vesikulärer Stomatitis, Rinderpest, infektiöser Pleuropneumonie der Rinder, Rifttalfieber, Schafpocken und Ziegenpocken, Afrikanischem Schweinefieber und hochpathogener Vogelgrippe verbunden ist, oder eine Variante von solch einer Komponente mit bis zu 5 % Substitutionen ist.

8. Verbindung nach einem der vorangehenden Ansprüche, wobei der Rest und das Antigen kovalent gebunden sind.

9. Verbindung nach einem der vorangehenden Ansprüche, wobei der Rest und das Antigen jeweils ein Polypeptid aufweisen und beide in derselben Polypeptidkette vorliegen.

10. Nukleinsäuremolekül, das eine Verbindung nach Anspruch 9 codiert.

11. Expressionsvektor, der ein Nukleinsäuremolekül nach Anspruch 10 aufweist.

12. Wirtszelle mit einem Nukleinsäuremolekühl nach Anspruch 10 oder einem Expressionsvektor nach Anspruch 11.

13. Impfstoff mit einer Verbindung nach einem der Ansprüche 1 bis 9 oder einem Nukleinsäuremolekül nach Anspruch 10.

14. Impfstoff nach Anspruch 13, der weiterhin einen Hilfsstoff aufweist.

15. Verbindung nach einem der Ansprüche 1 bis 9 oder Nukleinsäuremolekül nach Anspruch 10 zur Verwendung in der Medizin.

16. Pharmazeutische Zusammensetzung mit einer Verbindung nach einem der Ansprüche 1 bis 9 oder einem Nukleinsäuremolekül nach Anspruch 10 und einem pharmazeutisch akzeptablen Träger.

17. Verwendung einer Verbindung nach einem der Ansprüche 1 bis 9 oder Nukleinsäure nach Anspruch 10 bei der Herstellung eines Medikaments zum Bekämpfen einer Krankheit bei einem Tier.

18. Verwendung einer Verbindung nach einem der Ansprüche 1 bis 9 oder Nukleinsäure nach Anspruch 10 bei der Herstellung eines Impfstoffs zum Immunisieren eines Tiers.

19. Verwendung nach Anspruch 17 oder 18, wobei die Krankheit durch ein Pathogen verursacht wird.

20. Verwendung nach Anspruch 19, wobei das Pathogen ein Bakterium, Virus, Pilz, Protozoon oder Ethelminth ist.

21. Verwendung nach Anspruch 10, wobei das Pathogen ein solches ist, das mit Maul und Klauenseuche, vesikulärer Virusseuche der Schweine, Pest der kleinen Wiederkäuer, Dermatitis nodularis, Blauzungenkrankheit, Afrikanischer Pferdepest, klassischem Schweinefieber, Newcastle-Krankheit, vesikulärer Stomatitis, Rinderpest, infektiöser Pleuropneumonie der Rinder, Rifttalfieber, Schafpocken und Ziegenpocken, Afrikanischem Schweinefieber und hochpathogener Vogelgrippe verbunden ist.

22. Verwendung nach Anspruch 17 oder 18, wobei das Tier ein Säugetier ist.

23. Verwendung nach Anspruch 17 oder 18, wobei das Tier ein Haus- oder Nutztier ist.

24. Verwendung nach Anspruch 22 oder 23. wobei das Tier eine Kuh, ein Schaf, ein Pferd, ein Schwein, eine Ziege, ein Hund, eine Katze oder ein Kaninchen ist.

25. Verfahren zur Herstellung einer Verbindung nach Anspruch 1, das das Verknüpfen des Rests und des Antigens aufweist.

26. Verfahren zum Herstellen einer Verbindung nach Anspruch 9, die ein Polypeptid aufweist, wobei das Verfahren das (i) Kultivieren einer Wirtszelle nach Anspruch 12, die das Polypeptid exprimiert; und (ii) Isolieren des Polypeptids aufweist.

27. Verfahren zum Herstellen einer Nukleinsäure nach Anspruch 10, die das Verknüpfen eines Nukleinsäuremoleküls, das den Rest codiert, und eines Nukleinsäuremoleküls, das das Antigen codiert, aufweist.

28. Verfahren zum Bestimmen, ob einem Tier eine Verbindung nach Anspruch 1 verabreicht wurde, wobei das Verfahren das Bestimmen in einer von dem Tier genommenen Antikörperprobe aufweist, ob das Tier eine Immunantwort auf den Rest der Verbindung, wie er in Anspruch 1 definiert ist, gehabt hat.

29. Verfahren nach Anspruch 28, das den weiteren Schritt des Bestimmens aufweist, ob das Tier eine Immunantwort auf das Antigen gehabt hat, das in der Verbindung vorliegt.

30. Teilesatz mit (i) einer Verbindung nach einem der Ansprüche 1 bis 9 oder einem Nukleinsäuremolekül nach Anspruch 10 und (ii) Mitteln zum Bestimmen einer Immunantwort auf den Rest, der bei der Verbindung vorliegt, und/oder (iii) Mittel zum Bestimmen einer Immunantwort auf das Antigen, das bei der Verbindung vorliegt.

31. Teilesatz nach Anspruch 30, wobei - falls vorliegend - Teil (ii) den gesamten oder einen Anteil größer als 10 Aminosäurereste in der Länge von dem Rest aufweist, der an einen Antikörper bindet, der gegen den Rest erzeugt wurde, und Teil (iii) das gesamte oder einen Anteil von mindestens 5 Aminosäuren in der Länge von dem Antigen aufweist, das an einen Antikörper bindet, der gegen das Antigen erzeugt wurde.

32. Teilesatz mit (i) Mitteln zum Detektieren einer Immunantwort auf ein Tierkrankheitantigen und (ii) Mitteln zum Detektieren einer Immunantwort auf einen Rest, der ausgewählt ist aus HIV gp120, dem LAM-Protein von *Mycobacterium tuberculosis* oder einem Glykoprotein von Ebolavirus oder einem Teil davon, der an DC-SIGN an einer dendritischen Zelle anbindet und größer als 10 Aminosäuren in der Länge ist.

33. Teilesatz nach einem der Ansprüche 30 bis 32, wobei die Mittel zum Detektieren einer Immunantwort ein ELISA sind.

## Revendications

1. Composé pour la vaccination d'un animal, comprenant (i) un groupe choisi parmi le gp120 du VIH, la protéine LAM de *Mycobacterium tuberculosis* ou une glycoprotéine du virus d'Ebola, ou une partie de ceux-ci qui se lie à DC-SIGN sur une cellule dendritique et qui est supérieure à 10 acides aminés en longueur ; et (ii) un antigène.

2. Composé selon la revendication 1, dans lequel l'antigène est un polypeptide.

3. Composé selon l'une quelconque des revendications précédentes, dans lequel l'antigène est un constituant antigénique d'un pathogène ou d'une tumeur, ou un antigène de prion.

4. Composé selon la revendication 3, dans lequel l'antigène comprend deux ou plus parmi un constituant antigénique d'un pathogène ou d'une tumeur, ou un antigène de prion.

5. Composé selon la revendication 3 ou 4, dans lequel l'antigène est une variante antigénique d'un constituant antigénique ayant jusqu'à 5 % de substitutions.

6. Composé selon les revendications 3 à 5, dans lequel le pathogène est l'un quelconque parmi une bactérie, un virus, un champignon, un protozoaire ou un helminthe.

7. Composé selon la revendication 6, dans lequel l'antigène est un constituant antigénique d'un pathogène associé à la fièvre aphteuse, la maladie vésiculeuse du porc, la peste des petits ruminants, la maladie de la peau bosselée, la langue bleue, la peste équine africaine, la peste porcine classique, la maladie de Newcastle, la stomatite vésiculeuse, la peste bovine, la péripneumonie contagieuse bovine, la fièvre de la vallée de Rift, la variole ovine et la variole caprine, la peste porcine africaine et à l'influenza aviaire fortement pathogène ou une variante d'un tel constituant ayant jusqu'à 5 % de substitutions.

8. Composé selon l'une quelconque des revendications précédentes, dans lequel le groupe et l'antigène sont liés de façon covalente.

9. Composé selon l'une quelconque des revendications précédentes, dans lequel le groupe et l'antigène comprennent chacun un polypeptide et les deux sont présents dans la même chaîne polypeptidique.

10. Molécule d'acide nucléique codant un composé selon la revendication 9.

11. Vecteur d'expression comprenant une molécule d'acide nucléique selon la revendication 10.

12. Cellule hôte comprenant une molécule d'acide nucléique selon la revendication 10 ou un vecteur d'expression selon la revendication 11.

13. Vaccin comprenant un composé selon l'une quelconque des revendications 1 à 9 ou une molécule d'acide nucléique selon la revendication 10.

14. Vaccin selon la revendication 13, comprenant en outre un adjuvant.

15. Composé selon l'une quelconque des revendications 1 à 9, ou molécule d'acide nucléique selon la revendication 10, pour l'utilisation en médecine.

16. Composition pharmaceutique comprenant un composé selon l'une quelconque des revendications 1 à 9, ou une molécule d'acide nucléique selon la revendication 10 et un véhicule pharmaceutiquement acceptable.

17. Utilisation d'un composé selon l'une quelconque des revendications 1 à 9 ou d'un acide nucléique selon la revendication 10, dans la préparation d'un médicament pour combattre une maladie chez un animal.

18. Utilisation d'un composé selon l'une quelconque des revendications 1 à 9 ou d'un acide nucléique selon la revendication 10, dans la préparation d'un vaccin pour immuniser un animal.

19. Utilisation selon la revendication 17 ou 18, dans laquelle la maladie est une maladie provoquée par un pathogène.

20. Utilisation selon la revendication 19, dans laquelle le pathogène est l'un quelconque parmi une bactérie, un virus, un champignon, un protozoaire ou un helminthe.

21. Utilisation selon la revendication 20, dans laquelle le pathogène est un pathogène associé à la fièvre aphteuse, la maladie vésiculeuse du porc, la peste des petits ruminants, la maladie de la peau bosselée, la langue bleue, la peste équine africaine, la peste porcine classique, la maladie de Newcastle, la stomatite vésiculeuse, la peste bovine, la péripneumonie contagieuse bovine, la fièvre de la vallée de Rift, la variole ovine et la variole caprine, la peste porcine africaine et à l'influenza aviaire fortement pathogène.

22. Utilisation selon la revendication 17 ou 18, dans laquelle l'animal est un mammifère.

23. Utilisation selon la revendication 17 ou 18, dans laquelle l'animal est un animal de compagnie ou un animal de ferme.

24. Utilisation selon la revendication 22 ou 23, dans laquelle l'animal est une vache, un mouton, un cheval, un porc, une chèvre, un chien, un chat ou un lapin.

25. Procédé de préparation d'un composé selon la revendication 1, comprenant la liaison dudit groupe et dudit antigène.

26. Procédé de préparation d'un composé selon le revendication 9 qui comprend un polypeptide, ledit procédé comprenant (i) la mise en culture d'une cellule hôte selon la revendication 12 qui exprime ledit polypeptide et (ii) l'isolement dudit polypeptide.

27. Procédé de préparation d'un acide nucléique selon la revendication 10, comprenant la liaison d'une molécule d'acide nucléique qui code le groupe et d'une molécule d'acide nucléique qui code un antigène.

28. Procédé pour déterminer si un composé selon la revendication 1 a été administré à un animal, le procédé comprenant la détermination, dans un échantillon contenant des anticorps prélevé auprès de l'animal, du fait de savoir si l'animal a eu une réponse immunitaire vis-à-vis du groupe dans le composé tel que défini dans la revendication 1.

29. Procédé selon la revendication 28, comprenant l'étape supplémentaire consistant à déterminer si l'animal a eu une réponse immunitaire vis-à-vis de l'antigène présent dans ledit composé.

30. Kit d'éléments comprenant (i) un composé selon l'une quelconque des revendications 1 à 9 ou une molécule d'acide nucléique selon la revendication 10, et (ii) des moyens pour détecter une réponse immunitaire vis-à-vis du groupe présent dans ledit composé et/ou (iii) des moyens pour détecter une réponse immunitaire vis-à-vis de l'antigène présent dans ledit composé.

31. Kit d'éléments selon la revendication 30, dans lequel, si elle est présente, la partie (ii) comprend la totalité, ou une partie supérieure à 10 acides aminés en longueur, dudit groupe qui se lie à un anticorps dirigé contre ledit groupe, et la partie (iii) comprend la totalité, ou une partie d'au moins 5 acides aminés en longueur, dudit antigène qui se lie à un anticorps dirigé contre ledit antigène.

32. Kit d'éléments comprenant (i) des moyens pour détecter une réponse immunitaire vis-à-vis d'un antigène de maladie animale et (ii) des moyens pour détecter une réponse immunitaire vis-à-vis d'un groupe choisi parmi le gp120 du VIH, la protéine LAM de *Mycobacterium tuberculosis* ou une glycoprotéine du virus d'Ebola, ou une partie de ceux-ci qui se lie à DC-SIGN sur une cellule dendritique et qui est supérieure à 10 acides aminés en longueur.

33. Kit d'éléments selon l'une quelconque des revendications 30 à 32, dans lequel les moyens pour détecter une réponse immunitaire sont un ELISA.
